# EUROPEAN PATENT SPECIFICATION

(11) **EP 1 115 782 B1**
(45) Date of publication and mention of the grant of the patent: **26.03.2003**
(21) Application number: 99936806.1
(22) Date of filing: 29.07.1999
(51) Int. Cl.: C08K 5/00, A61L 2/00

(54) **METHOD OF PREPARING A STERILE ARTICLE**
VERFAHREN ZUR HERSTELLUNG EINES STERILEN ARTIKELS
PROCEDE DE PRODUCTION D'UN ARTICLE STERILE

(30) Priority: 17.08.1998 US 135120
(43) Date of publication of application: 18.07.2001
(73) Proprietor: OCCIDENTAL CHEMICAL CORPORATION, Dallas, TX 75244-6119 (US)
(72) Inventor: WANG, Qi, Grand Island, NY 14072 (US); NAGY, Sandor, Mason, Ohio 45040 (US); XU, Mingzhu, Amherst, NY 14228 (US)
(74) Representative: Frankland, Nigel Howard
(86) International application number: GB9902484
(87) International publication number: WO00009600

(56) References cited:
- EP-A- 0 345 662
- FR-A- 1 546 264

## Description

This invention relates to the preparation of sterile articles.

Poly(vinyl chloride) (PVC) has properties that make it desirable for use as medical devices and as packaging for medical applications. Articles that are used in the medical field have to be sterilized before they are used. Gamma radiation is often used for this purpose. It has been found, however, that gamma radiation causes PVC to become yellow and medical articles that have turned yellow may be rejected as being of inferior quality. Articles made of PVC that are re-used and sterilized after each use are especially likely to turn yellow.

A variety of substances have been added to PVC to stabilize it from gamma radiation and prevent yellowing, including organic Ca\Zn soap blends (JP 57,003,653), organo tin compounds (JP 02189352, JP 08073619, and EP 83112278), and sulfur compounds (JP 08151495, 07102142, and 01278551).

According to one aspect of the present invention there is provided a method of preparing a sterile article comprising
(A) producing a chlorinated polymer selected from polyvinyl chloride and polyvinylidene chloride that contains about 0.0001 to about 5 wt% of a zinc complex having at least one anionic ligand that has at least one resonance structure in which the charge is localized on a carbon atom;
(B) fabricating said article from said chlorinated polymer; and
(C) sterilizing said article with gamma radiation.

Preferably said zinc complex has the general formula. 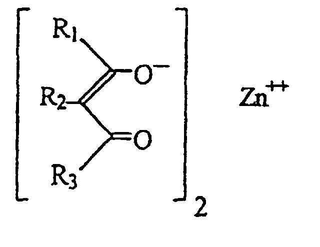 wherein R₁, R₂, and R₃ are each independently selected from R, OR, SR, and N(R)₂, R is hydrogen, alkyl from C₁ to C₁₂, or aryl from C₆ to C₁₈.

Advantageously R₁ and R₃ are each independently selected from alkyl and alkoxide from C₁ to C₈.

Conveniently R₂ is hydrogen.

Conveniently said metal complex has the formula

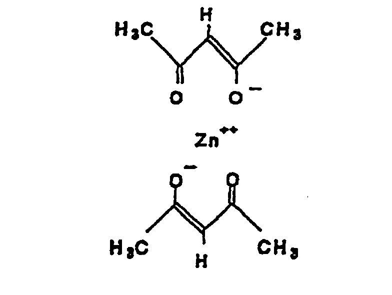

Preferably said metal complex is selected from

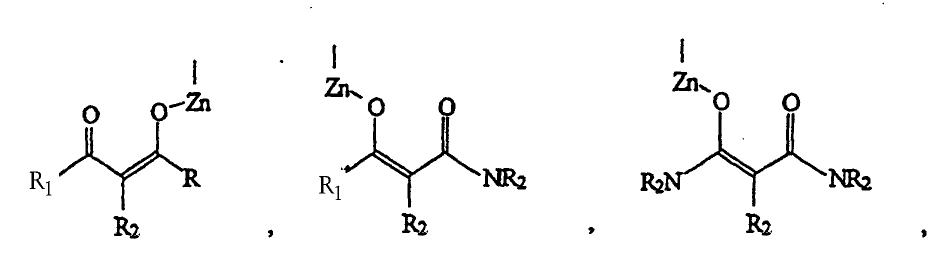

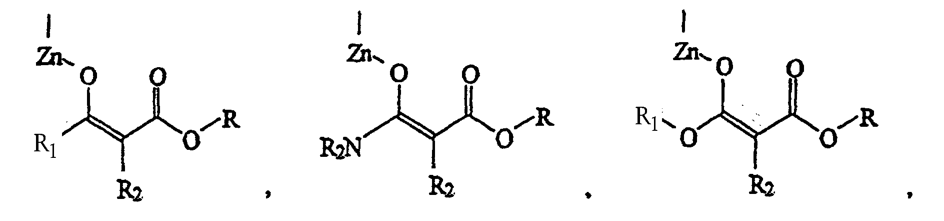

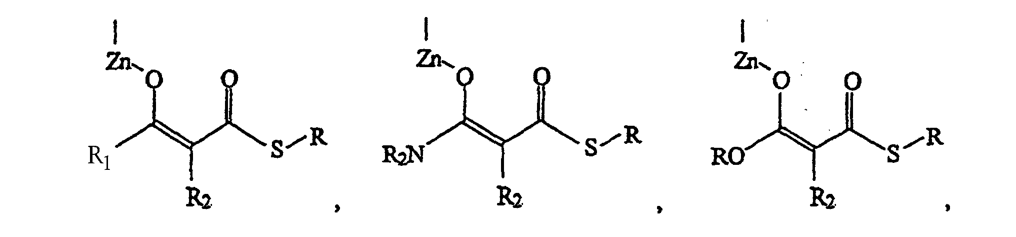

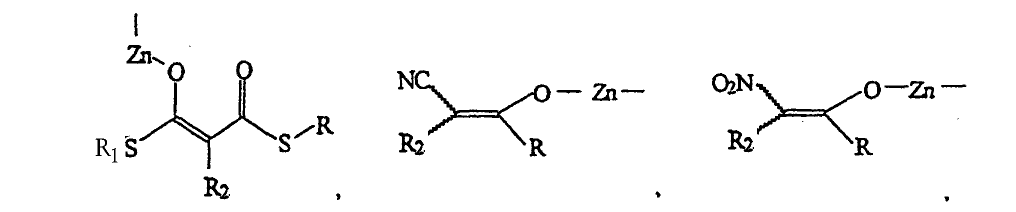

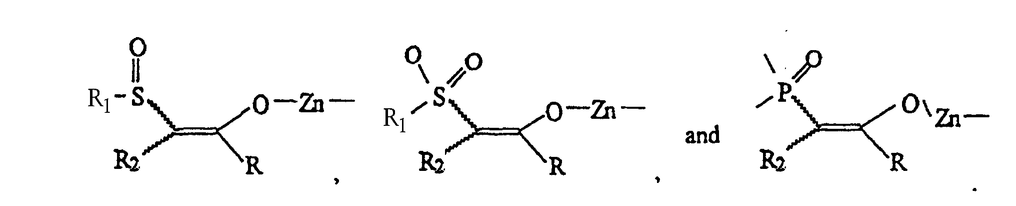

Advantageously said chlorinated polymer is poly(vinyl chloride).

Conveniently said chlorinated polymer is polyvinylidene chloride.

According to another aspect of the present invention there is provided a method of preparing a sterile article comprising
(A) making poly(vinyl chloride) that contains about 0.001 to about 0.5 wt% of a zinc complex having the general formula 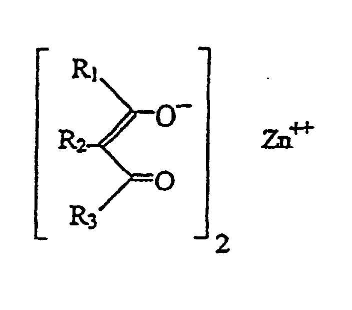 where R₁, R₂, and R₃ are each independently selected from R, OR, SR, and N(R)₂, and R is hydrogen, alkyl from C₁ to C₁₂, or aryl from C₆ to C₁₈;
(B) fabricating said article from said poly(vinyl chloride); and
(C) repeatedly sterilizing said article with gamma radiation.

Preferably said zinc complex has the formula 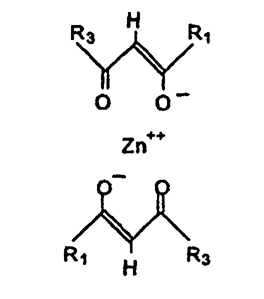 where R₁ and R₃ are each independently selected from alkyl and alkoxide from C₁ to C₈.

Advantageously R₁ and R₃ are CH₃.

Conveniently said zinc complex is selected from

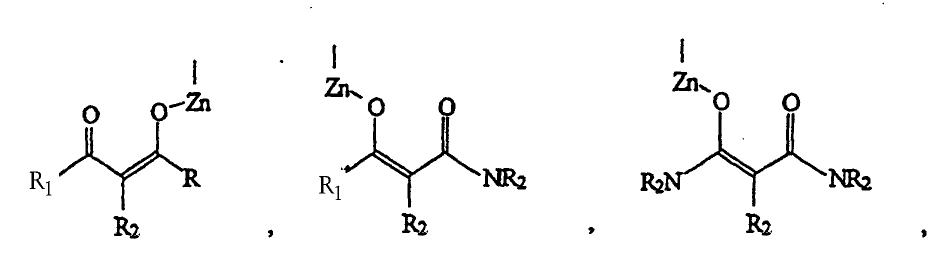

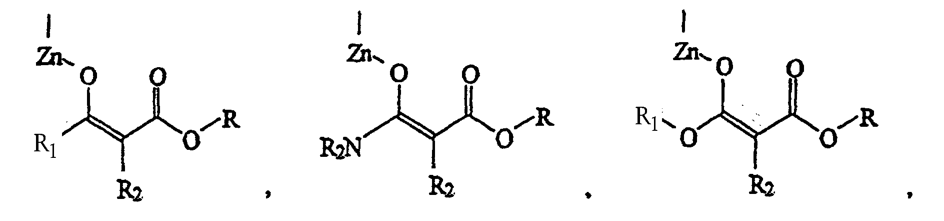

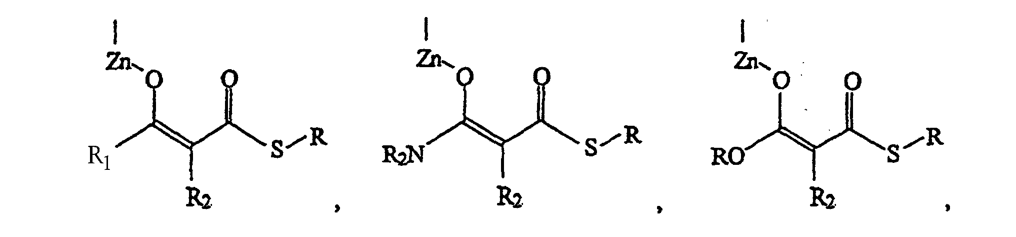

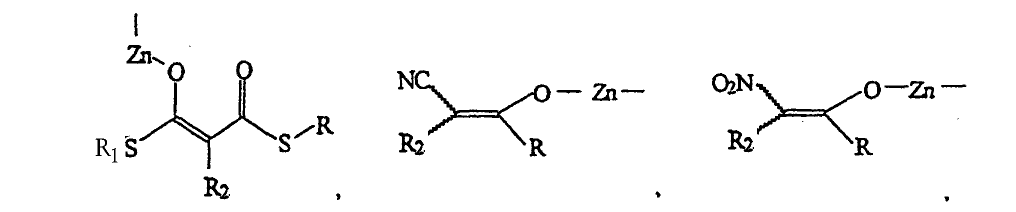

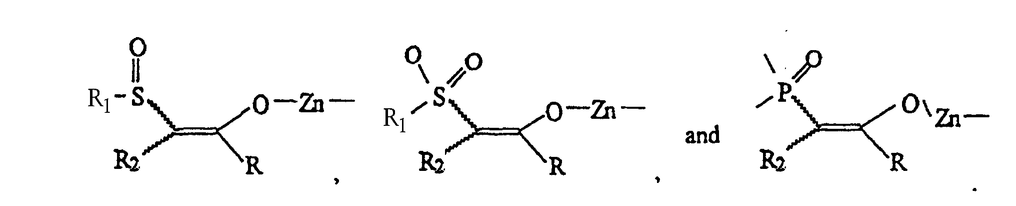

According to a further alternative embodiment of the present invention there is provided a method of preparing a sterile article comprising
(A) making poly(vinyl chloride) that contains about 0.001 to about 0.5 wt% of a zinc complex having the general formula 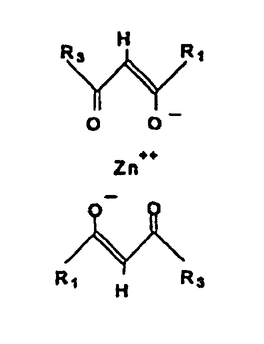 where R₁ and R₃ are each independently selected from alkyl and alkoxide from C₁ to C₈;
(B) fabricating said article from said poly(vinyl chloride); and
(C) repeatedly sterilizing said article with gamma radiation.

Preferably R₁ and R₃ are methyl.

According to a further preferred embodiment of the present invention there is provided a sterile article prepared according to any of the methods described above.

The article may be a medical article.

The addition of certain zinc complexes to PVC and polyvinylidene chloride has been found to stabilize the polymers against gamma radiation. The zinc complexes can be easily added to the polymers during polymerization or processing so that no additional procedural steps are required. The articles can be sterilized with gamma radiation without significant yellowing.

In the method of this invention, a sterile medical article or sterile packaging is prepared by first producing a chlorinated polymer with a zinc complex additive. The chlorinated polymer may be either PVC or polyvinylidene chloride. The zinc complex can be added to the polymer at any stage, such as, for example, to the monomer, to the partially polymerized monomer, during the drying of the polymer, or during processing of the polymer into the medical article. However, it is preferably added at the earliest possible stage after the monomer has at least partially polymerized in order to avoid any extra procedural steps and to ensure its complete incorporation into the polymer.

The zinc complex that is added to the polymer to stabilize it against gamma radiation contains at least one anionic ligand that has at least one resonance structure in which the charge is localized on a carbon atom. That is, the anionic ligand resonates between two structures and, in at least one of those structures, the negative charge is localized on a carbon atom. For example, if the anionic ligand is acetylacetonate the following two structures resonate, where the negative charge is on the carbon atom in one of the two structures.

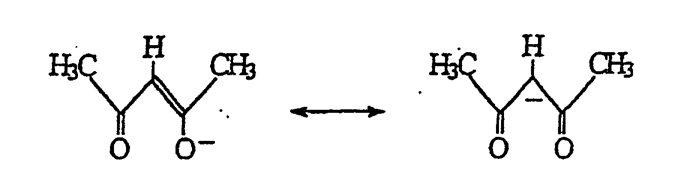

Generally, the zinc complex can have the general formula 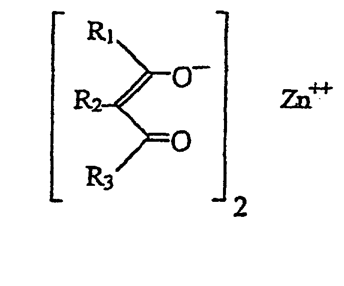 where R₁, R₂, and R₃ are each independently selected from R, OR, SR, and N(R)₂, and where R is hydrogen, alkyl from C₁ to C₁₂, or aryl from C₆ to C₁₈. Preferably, R₁ and R₃ are each independently selected from alkyl and alkoxide from C₁ to C₈ and are the same as that simplifies synthesis of the complex. Preferably, R₂ is hydrogen as those compounds are more readily available. Zinc is the preferred metal as zinc complexes have less toxicity and are less expensive. The Lewis acidity of those complexes is in the desirable range and adjustments to acidity can be

Examples of more preferred zinc complexes include.

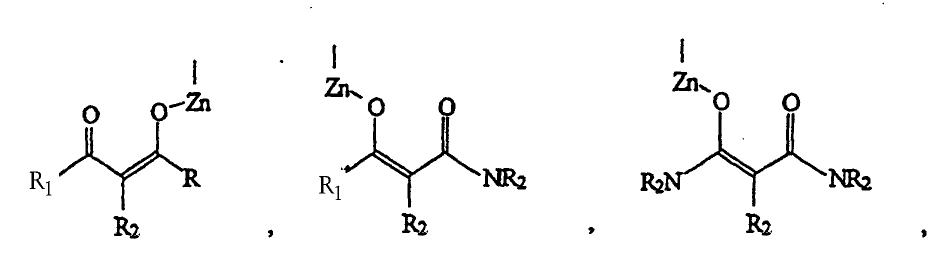

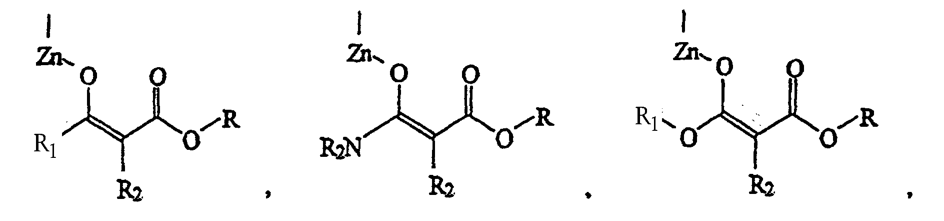

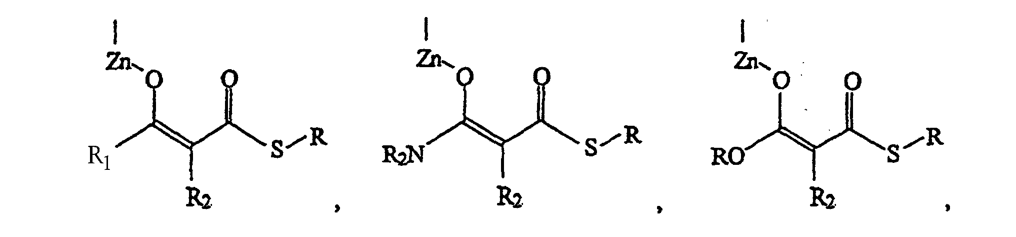

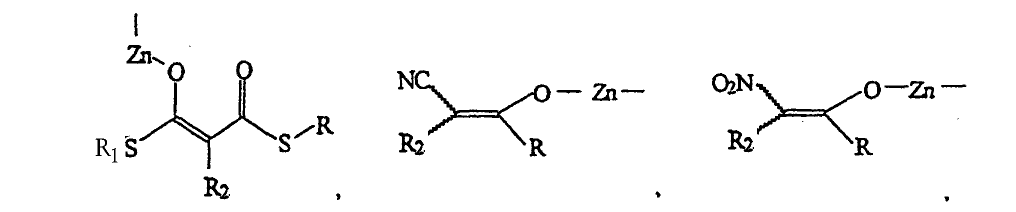

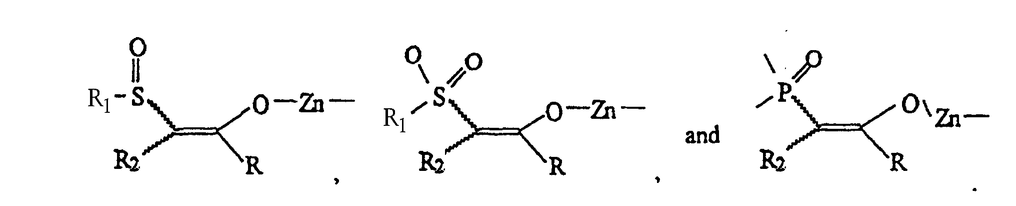

The amount of zinc complex added can be about 0.0001 wt% to about 5 wt%, based on the weight of the polymer; the preferred range is about 0.001 to about 0.5 wt%.

In addition, plasticizers, heat stabilizers, epoxidized soybean oil, lubricants, and other additives can be mixed in with the PVC as is well known in the art.

The following examples further illustrate this invention.

### Examples 1 to 5

To mixtures of 150.00 g PVC (sold by Occidental Chemical Corp. as "Oxy 240"), 0.30 g stearic acid, 0.23 g of a soap thermal stabilizer sold by Witco as "Mark 152 S," 97.50 g dioctyl phthalate, and 15.00 g epoxidized soybean oil sold by Witco as "Drapex 6.8" was added various amounts of different zinc salts of β-biscarbonyl compounds. The mixtures were thoroughly blended and hot milled at 300°F (149°C) for 5 minutes. The resulting PVC sheets were pressed at 330°F (166°C) and cut into 4''x3''x1/4" (10 cm x 8 cm x 0.6 cm) plaques. The plaques were divided into two smaller pieces. One piece was saved for the purpose of comparison and the other one was subjected to 5.0 Mrad of γ radiation at 50 kGy. The irradiated piece was again divided into two pieces and one piece was oven aged at 50°C for 48 hours. All three pieces were measured for yellowness index with a Mascbeth 2020 Plus Color Eye Spectrometer. The control materials were similarly prepared, but without a zinc complex of a β-biscarbonyl compound. The following table gives the results:

| Example | Metal Complex | Yellowness | | |
|---|---|---|---|---|
| | | With Metal Complex | | Without Metal Complex |
| 1 | 0.35 g Zn acetylacetonate | Initial | 11.3 | 12.4 |
| | | After γ | 29.4 | 45.0 |
| | | Aged | 37.2 | 72.0 |
| 2 | 0.60 g Zn acetylacetonate | Initial | 21.6 | 19.8 |
| | | After γ | 34.1 | 52.8 |
| | | Aged | 38.8 | 73.3 |
| 3 | 0.91 g Zn acetylacetonate | Initial | 34.5 | 19.8 |
| | | After γ | 35.1 | 52.8 |
| | | Aged | 37.4 | 73.3 |
| 4 | 0.35 g Zn bis(2,2,6,6-tetramethyl-3,5-heptanedionate | Initial | 16.4 | 12.4 |
| | | After γ | 39.5 | 45.0 |
| | | Aged | 57.7 | 72.0 |
| 5 | 0.31 g Zn hexafluoroacetylacetonate | Initial | 11.6 | 12.4 |
| | | After γ | 36.8 | 45.0 |
| | | Aged | 55.0 | 72.0 |

The table shows that the PVC that contained zinc salts of β-biscarbonyl compounds had significantly less discoloration after γ radiation compared with the control samples, which did not contain those compounds.

### Examples 6 to 9

Example 1 was repeated using various zinc salts as γ radiation stabilizers or the same molar equivalent amount of the corresponding ketone (see JP 02263853). The following table gives the stabilizers tested and the results:

| | | Yellowness | | |
|---|---|---|---|---|
| Example | Zinc Salt (g) Ketone | Initial | After γ Rays | After Aging |
| 6 | Zinc Acetylacetonate (0.40) | 19.1 | 38.6 | 45.9 |
| | 2,4-pentanedione | 16.9 | 55.8 | 76.3 |
| 7 | Zinc 1-benzoylacetonate (0.59) | 18.9 | 39.4 | 44.9 |
| | 1-benzoylacetone | 15.6 | 50.9 | 72.6 |
| 8 | Zinc(methoxyethoxy)carbonyl acetonate (0.58) | 19.0 | 40.6 | 49.1 |
| | 2-methoxyethoxy acetoacetate | 18.0 | 54.0 | 73.3 |
| 9 | Zinc 3-(2'-ethytcarboxyethyl)-2,4-pentanedionate (0.70) | 21.9 | 45.0 | 52.9 |
| | Ethyl 4-acetyl-5-oxohexanoate | 17.1 | 49.6 | 61.5 |

The above table shows that the zinc salts are more effective in reducing yellowness than the corresponding ketones.

In the present specification the word "comprise" or "comprises means "includes or consists of" and "comprising" means "including or consisting of

## Claims

1. A method of preparing a sterile article comprising
(A) producing a chlorinated polymer selected from polyvinyl chloride and polyvinylidene chloride that contains about 0.0001 to about 5 wt% of a zinc complex having at least one anionic ligand that has at least one resonance structure in which the charge is localized on a carbon atom;
(B) fabricating said article from said chlorinated polymer; and
(C) sterilizing said article with gamma radiation.

2. A method according to Claim 1 wherein said metal complex has the general formula 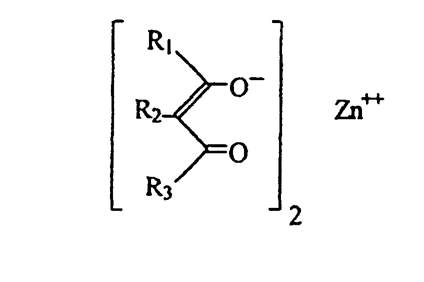 wherein R₁, R₂, and R₃ are each independently selected from R, OR, SR, and N(R)₂, R is hydrogen, alkyl from C₁ to C₁₂, or aryl from C₆ to C₁₈.

3. A method according to Claim 2 wherein R₁ and R₃ are each independently selected from alkyl and alkoxide from C₁ to C₈.

4. A method according to Claim 2 or Claim 3 wherein R₂ is hydrogen.

5. A method according to Claim 4 wherein metal complex has the formula

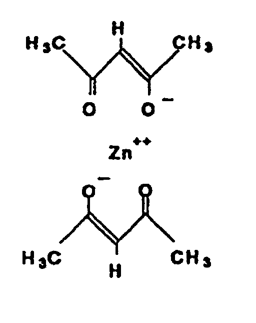

6. A method according to Claim 2 wherein said metal complex is selected from

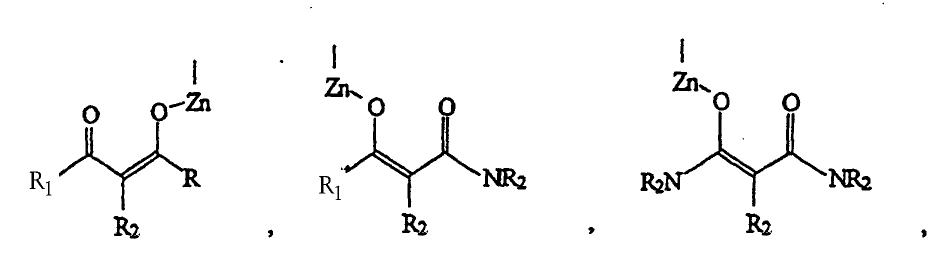

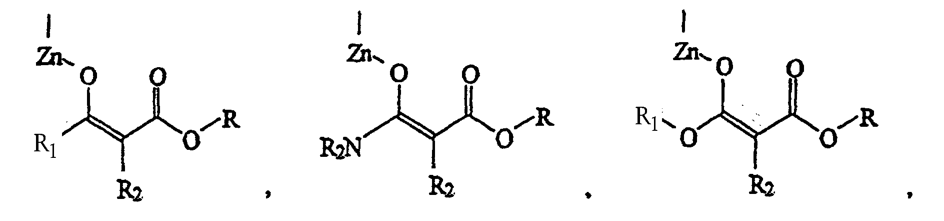

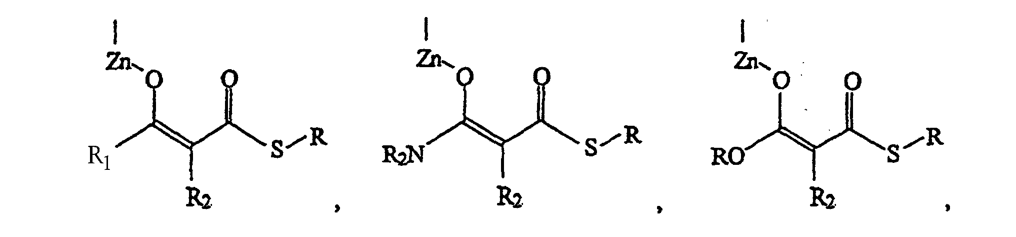

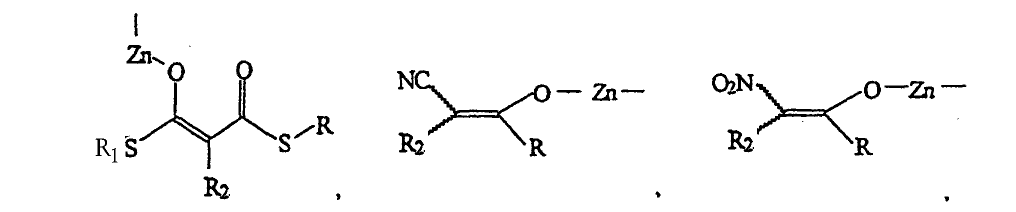

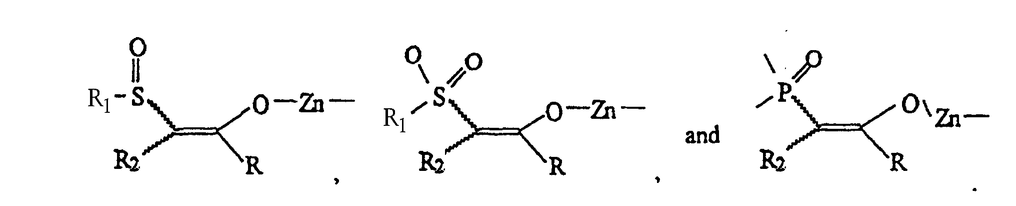

7. A method according to any one of the preceding claims wherein said chlorinated polymer is poly(vinyl chloride).

8. A method according to any one of the preceding claims wherein said chlorinated polymer is polyvinylidene chloride.

9. A method of preparing a sterile article comprising
(A) making poly(vinyl chloride) that contains about 0.001 to about 0.5 wt% of a zinc complex having the general formula 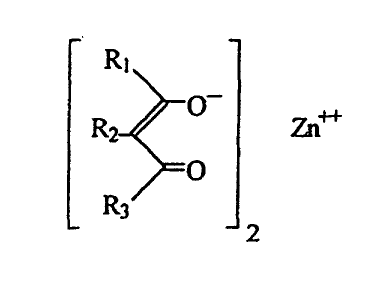 where R₁, R₂, and R₃ are each independently selected from R, OR, SR, and N(R)₂, and R is hydrogen, alkyl from C₁ to C₁₂, or aryl from C₆ to C₁₈;
(B) fabricating said article from said poly(vinyl chloride); and
(C) repeatedly sterilizing said article with gamma radiation.

10. A method according to Claim 9 wherein said zinc complex has the formula 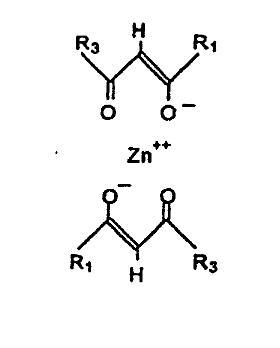 where R₁ and R₃ are each independently selected from alkyl and alkoxide from C₁ to C₈.

11. A method according to Claim 9 or Claim 10 wherein R₁ and R₃ are CH₃.

12. A method according to Claim 9 wherein said zinc complex is selected from

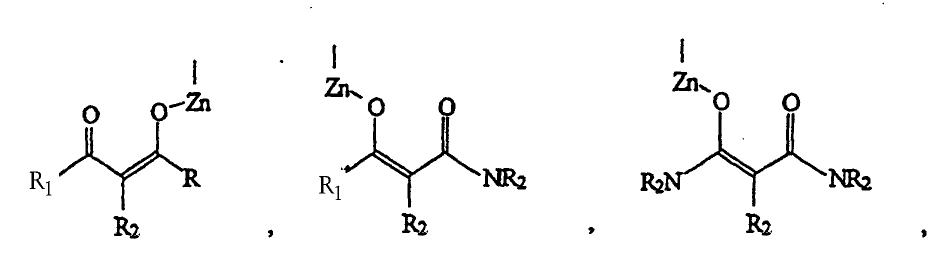

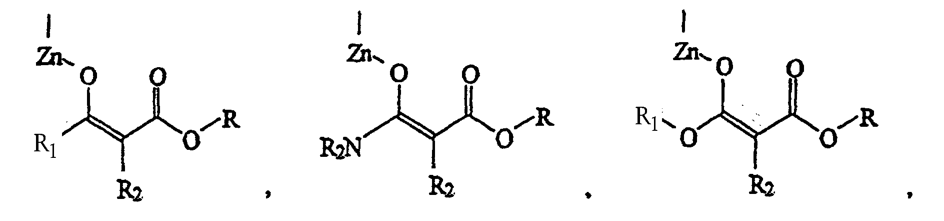

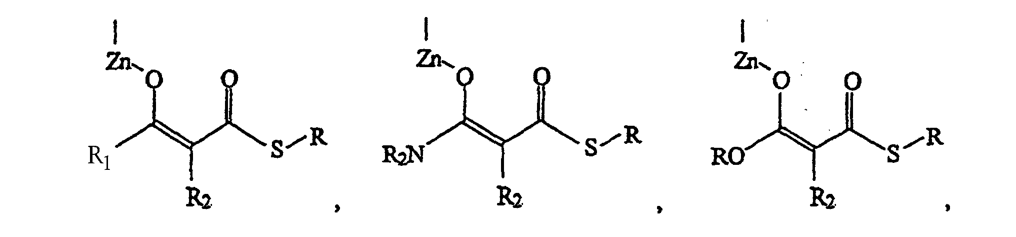

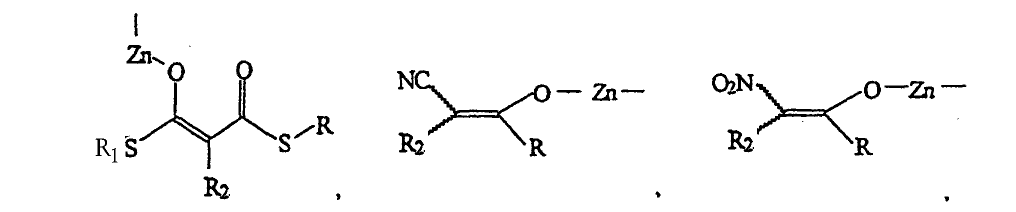

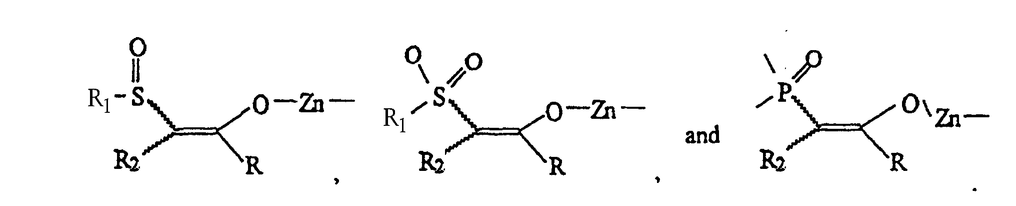

13. A method of preparing a sterile article comprising
(A) making poly(vinyl chloride) that contains about 0.001 to about 0.5 wt% of a zinc complex having the general formula 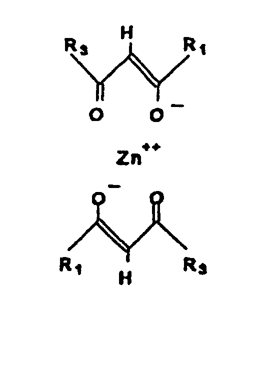 where R₁ and R₃ are each independently selected from alkyl and alkoxide from C₁ to C₈;
(B) fabricating said article from said poly(vinyl chloride); and
(C) repeatedly sterilizing said article with gamma radiation.

14. A method according to Claim 13 wherein R₁ and R₃ are methyl.

15. A sterile article prepared according to the method of any one of the preceding Claims.

## Patentansprüche

1. Verfahren zur Herstellung eines sterilen Artikels, welches umfaßt:
(A) Herstellen eines chlorierten Polymers, das ausgewählt wird aus Polyvinylchlorid und Polyvinylidenchlorid, und welches etwa 0,0001 bis etwa 5 Gew.-% eines Zinkkomplexes enthält, welcher wenigstens einen anionischen Liganden aufweist, der wenigstens eine Resonanzstruktur aufweist, in welcher die Ladung an einem Kohlenstoffatom lokalisiert ist;
(B) Herstellen des Artikels aus dem chlorierten Polymer; und
(C) Sterilisieren des Artikels mit Gamma-Strahlung.

2. Verfahren nach Anspruch 1, **dadurch gekennzeichnet, daß** der Metallkomplex die allgemeine Formel 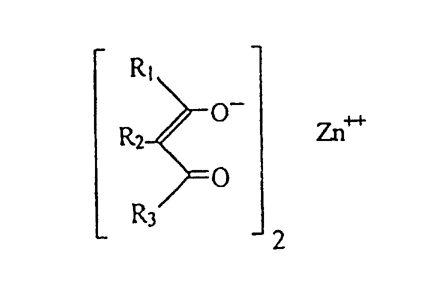 aufweist, wobei R₁, R₂ und R₃ jeweils unabhängig ausgewählt werden aus R, OR, SR und N(R)₂, wobei R Wasserstoff, Alkyl von C₁ bis C₁₂ oder Aryl von C₆ bis C₁₈ ist.

3. Verfahren nach Anspruch 2, **dadurch gekennzeichnet, daß** R₁ und R₃ jeweils unabhängig ausgewählt werden aus Alkyl und Alkoxid von C₁ bis C₈.

4. Verfahren nach Anspruch 2 oder Anspruch 3, **dadurch gekennzeichnet, daß** R₂ Wasserstoff ist.

5. Verfahren nach Anspruch 4, **dadurch gekennzeichnet, daß** der Metallkomplex die Formel 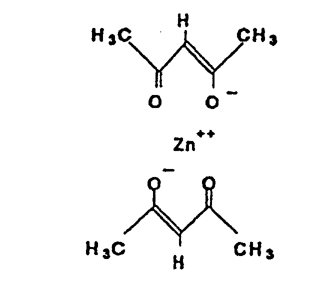 aufweist.

6. Verfahren nach Anspruch 2, **dadurch gekennzeichnet, daß** der Metallkomplex ausgewählt wird aus

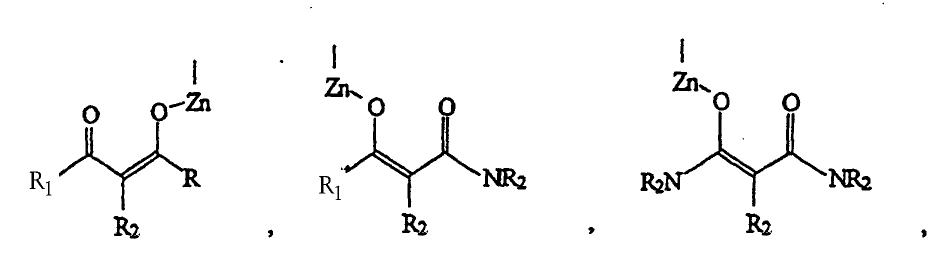

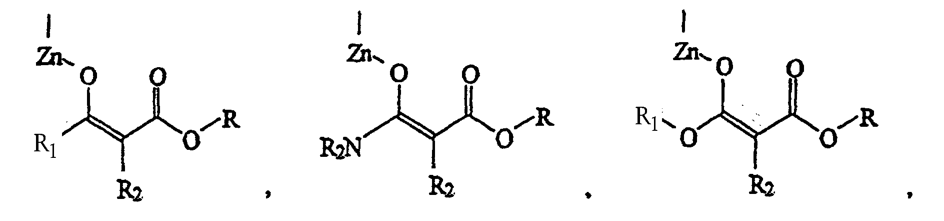

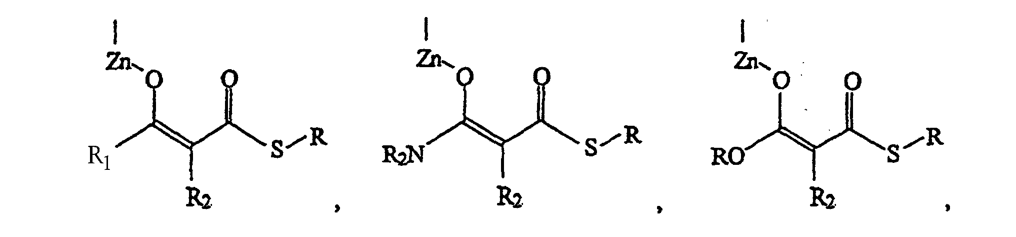

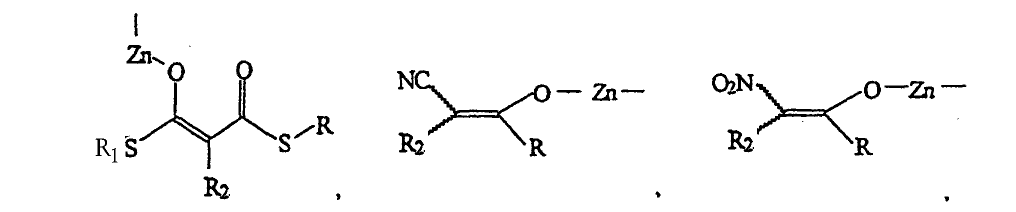

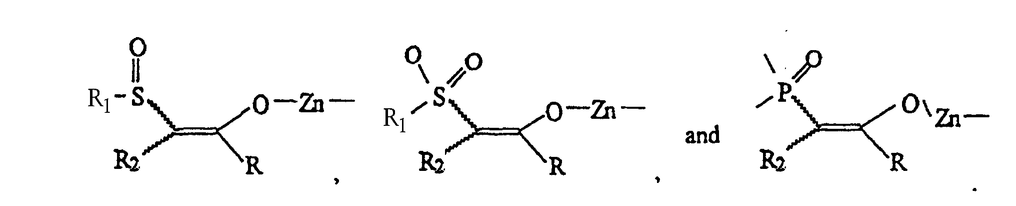

7. Verfahren nach einem der vorangehenden Ansprüche, **dadurch gekennzeichnet, daß** das chlorierte Polymer Poly(vinylchlorid) ist.

8. Verfahren nach einem der vorangehenden Ansprüche, **dadurch gekennzeichnet, daß** das chlorierte Polymer Polyvinylidenchlorid ist.

9. Verfahren zum Herstellen eines sterilen Artikels, welches umfaßt:
(A) Herstellen von Poly(vinylchlorid), welches etwa 0,001 bis etwa 0,5 Gew.-% eines Zinkkomplexes mit der allgemeinen Formel 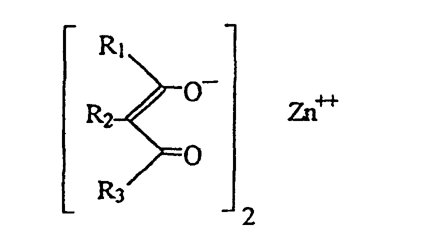 enthält, wobei R₁, R₂ und R₃ jeweils unabhängig ausgewählt werden aus R, OR, SR und N(R)₂, und wobei R Wasserstoff, Alkyl von C₁ bis C₁₂ oder Aryl von C₆ bis C₁₈ ist;
(B) Herstellen des Artikels aus dem Poly(vinylchlorid); und
(C) wiederholtes Sterilisieren des Artikels mit Gamma-Strahlung.

10. Verfahren nach Anspruch 9, **dadurch gekennzeichnet, daß** der Zinkkomplex die Formel 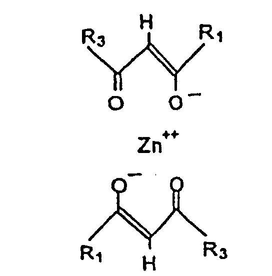 aufweist, wobei R₁ und R₃ jeweils unabhängig ausgewählt werden von Alkyl und Alkoxid von C₁ bis C₈.

11. Verfahren nach Anspruch 9 oder Anspruch 10, **dadurch gekennzeichnet, daß** R₁ und R₃ CH₃ sind.

12. Verfahren nach Anspruch 9, **dadurch gekennzeichnet, daß** der Zinkkomplex ausgewählt wird aus

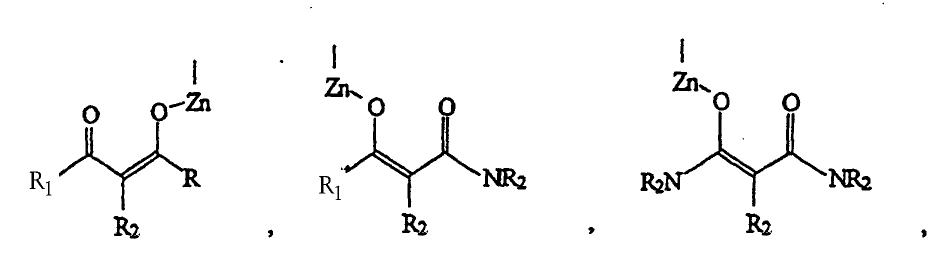

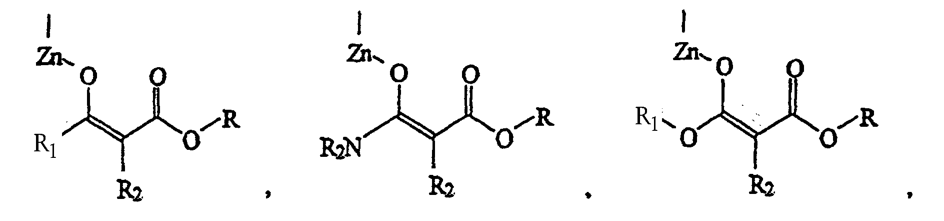

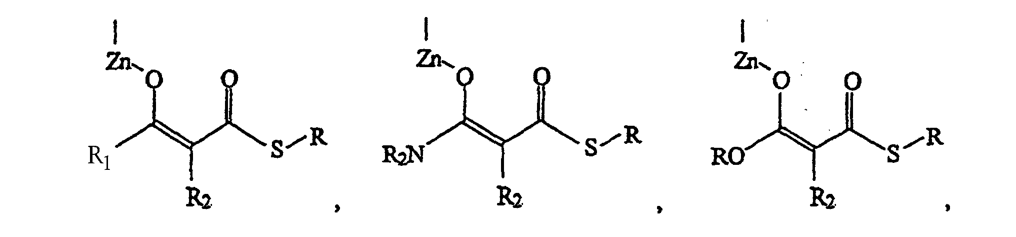

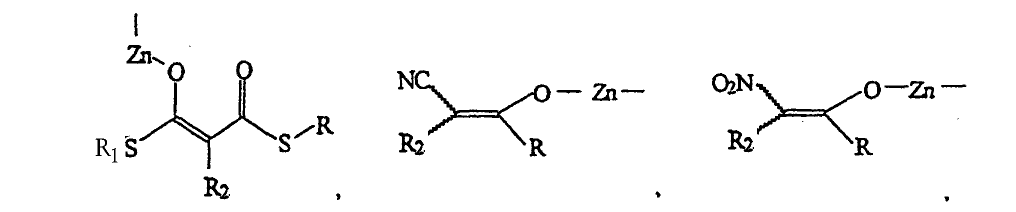

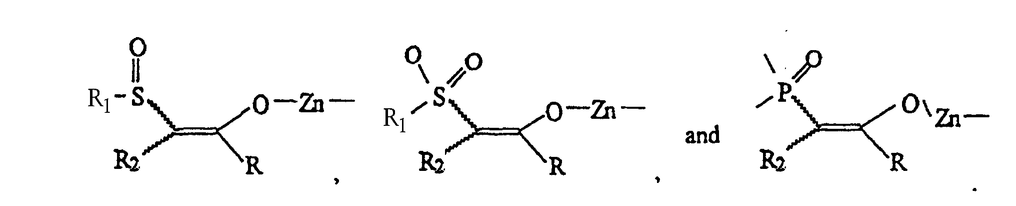

13. Verfahren zum Herstellen eines sterilen Artikels, welches umfaßt:
(A) Herstellen von Poly(vinylchlorid), welches etwa 0,001 bis etwa 0,5 Gew.-% eines Zinkkomplexes mit der allgemeinen Formel 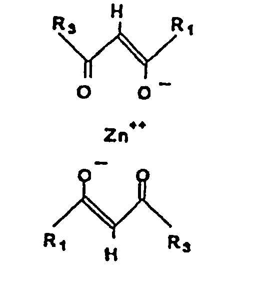 enthält, wobei R₁ und R₃ jeweils unabhängig ausgewählt werden aus Alkyl und Alkoxid von C₁ bis C₈;
(B) Herstellen des Artikels aus dem Poly(vinylchlorid); und
(C) wiederholtes Sterilisieren des Artikels mit Gamma-Strahlung.

14. Verfahren nach Anspruch 13, **dadurch gekennzeichnet, daß** R₁ und R₃ Methyl sind.

15. Steriler Artikel, der gemäß dem Verfahren nach einem der vorangehenden Ansprüche hergestellt ist.

## Revendications

1. Procédé de préparation d'un article stérile comprenant
(A) la production d'un polymère chloré choisi parmi le poly(chlorure de vinyle) et le poly(chlorure de vinylidène) qui contient environ 0,0001 à environ 5 % en poids d'un complexe de zinc comportant un ligand anionique qui possède au moins une structure de résonance dans laquelle la charge est localisée sur un atome de carbone ;
(B) la fabrication dudit article à partir dudit polymère chloré ; et
ICI la stérilisation dudit article avec un rayonnement gamma.

2. Procédé selon la revendication 1, dans lequel ledit complexe métallique répond à la formule générale 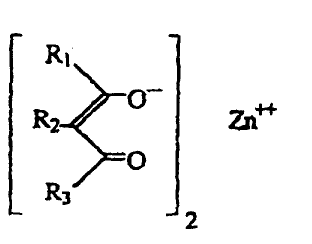 dans laquelle R₁, R₂ et R₃ sont chacun choisis indépendamment parmi R, OR, SR et N(R)₂, R est un atome d'hydrogène, un groupe alkyle en C₁-C₁₂ ou aryle en C₆-C₁₈.

3. Procédé selon la revendication 2, dans lequel R₁ et R₃ sont chacun choisis indépendamment parmi un groupe alkyle et alkylate en C₁-C₈.

4. Procédé selon la revendication 2 ou la revendication 3, dans lequel R₂ est un atome d'hydrogène.

5. Procédé selon la revendication 4, dans lequel le complexe métallique répond à la formule générale

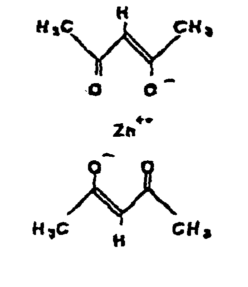

6. Procédé selon la revendication 2, dans lequel ledit complexe métallique est choisi parmi

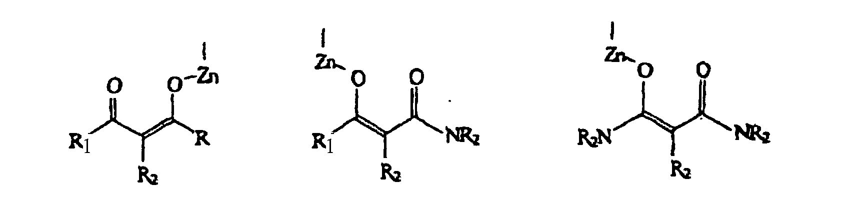

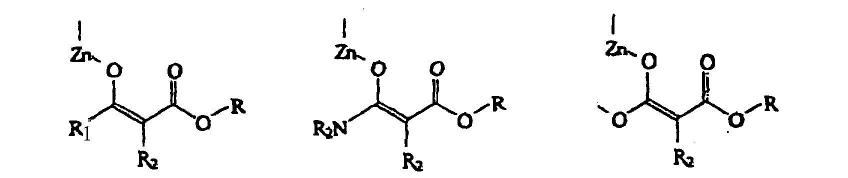

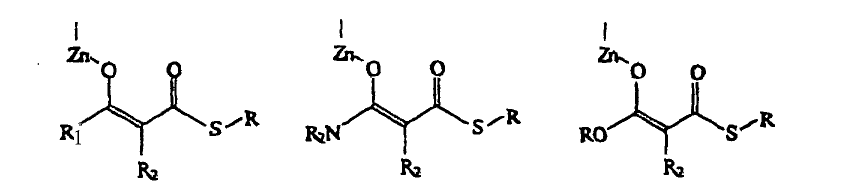

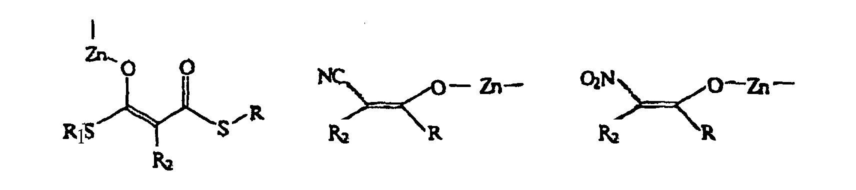

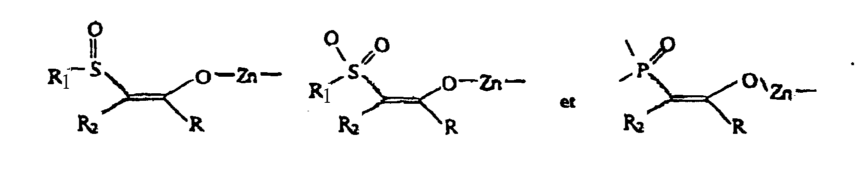

7. Procédé selon l'une quelconque des revendications précédentes, dans lequel ledit polymère chloré est le poly(chlorure de vinyle).

8. Procédé selon l'une quelconque des revendications précédentes, dans lequel ledit polymère chloré est le poly(chlorure de vinylidène).

9. Procédé de préparation d'un article stérile comprenant
(A) la fabrication de poly(chlorure de vinyle) qui contient environ 0,001 à environ 0,5 % en poids d'un complexe de zinc répondant à la formule générale 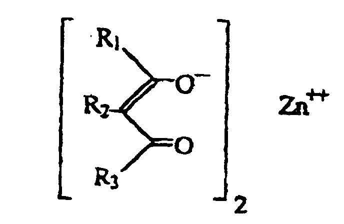 dans laquelle R₁, R₂ et R₃ sont chacun choisis indépendamment parmi R, OR, SR et N(R)₂, et R est un atome d'hydrogène, un groupe alkyle en C₁-C₁₂ ou aryle en C₆-C₁₈ ;
(B) la fabrication dudit article à partir dudit poly(chlorure de vinyle) ; et
(C) la stérilisation répétée dudit article avec un rayonnement gamma.

10. Procédé selon la revendication 9, dans lequel ledit complexe de zinc répond à la formule 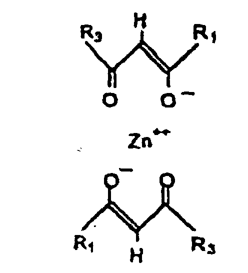 dans laquelle R₁ et R₃ sont chacun choisis indépendamment parmi un groupe alkyle et alkylate en C₁-C₈.

11. Procédé selon la revendication 9 ou la revendication 10, dans lequel R₁ et R₃ sont CH₃.

12. Procédé selon la revendication 9, dans lequel ledit complexe de zinc est choisi parmi

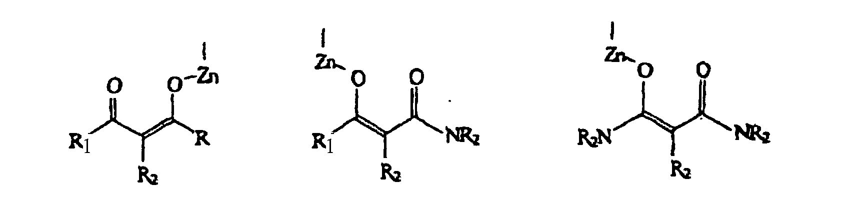

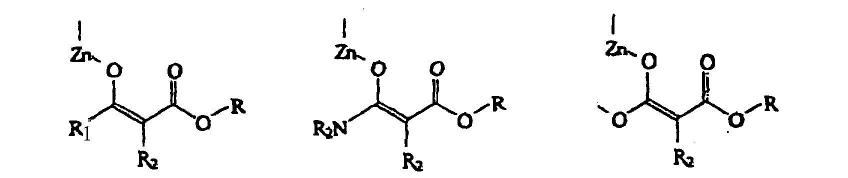

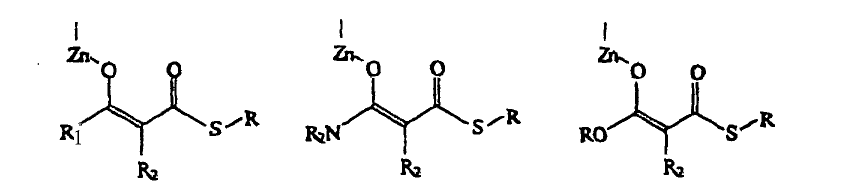

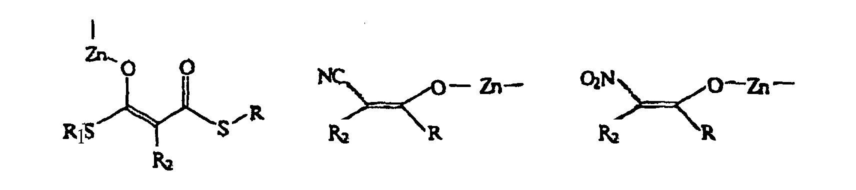

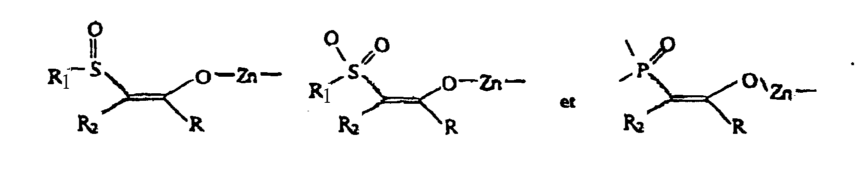

13. Procédé de préparation d'un article stérile comprenant
(A) la fabrication de poly(chlorure de vinyle) qui contient environ 0,001 à environ 0,5 % en poids d'un complexe de zinc répondant à la formule générale 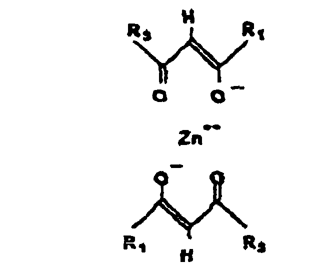 dans laquelle R₁ et R₃ sont chacun choisis indépendamment parmi un groupe alkyle ou alkylate en C₁-C₈ ;
(B) la fabrication dudit article à partir dudit poly(chlorure de vinyle) ; et
(C) la stérilisation répétée dudit article avec un rayonnement gamma.

14. Procédé selon la revendication 13, dans lequel R₁ et R₃ sont des groupes méthyle.

15. Article stérile préparé selon le procédé de l'une quelconque des revendications précédentes.
